# EUROPÄISCHE PATENTSCHRIFT

(11) **EP 0 687 671 B1**
(45) Veröffentlichungstag und Bekanntmachung des Hinweises auf die Patenterteilung: **10.09.1997**
(21) Anmeldenummer: 95108486.2
(22) Anmeldetag: 02.06.1995
(51) Int. Cl.: C07C 313/14, C07C 319/24, C07C 319/06, C07C 323/09

(54) **Verfahren zur Herstellung von 4-Fluorthiophenol**
Method for the preparation of 4-fluorothiophenol
Procédé pour la préparation de 4-fluorothiophénol

(30) Priorität: 15.06.1994 DE 4420777
(43) Veröffentlichungstag der Anmeldung: 20.12.1995
(73) Patentinhaber: BAYER AG, 51368 Leverkusen (DE)
(72) Erfinder: Fiege, Helmut, Dr., D-51373 Leverkusen (DE); Hagedorn, Ferdinand, Dr., D-51381 Leverkusen (DE); Eymann, Wolfgang, Dr., D-51061 Köln (DE); Neuner, Otto, Dr., D-51465 Bergisch Gladbach (DE); Müller, Herbert, Dr., D-52372 Kreuzau (DE)

(56) Entgegenhaltungen:
- DE-A- 1 816 902
- DE-A- 3 302 647
- DE-A- 3 304 054
- DE-A- 4 022 477
- CHEMICAL ABSTRACTS, vol. 51, no. 22, 25.November 1957 Columbus, Ohio, US; abstract no. 17793d, R. NODZU, ET AL.: 'Aromatic fluorine compounds. I. Preparations of p-fluoro- benzenesulphonic acid and related compounds' & NIPPON KAGAKU ZASSHI, 1955, 76, 775-778
- SYNTHETIC COMMUNICATIONS, Bd. 16, Nr. 7, 1986 NEW YORK, US, Seiten 819-825, A. OOKAWA, ET AL.: 'Chemoselective reduction of diaryl disulphides to thiols with sodium borohydride in mixed solvent containing methanol'

## Beschreibung

Die vorliegende Erfindung betrifft ein Verfahren mit einer speziellen Maßnahmenkombination zur Herstellung von reinem 4-Fluorthiophenol mit hoher Ausbeute.

Es ist bekannt 4-Fluorthiophenol durch Reduktion von 4-Fluorbenzolsulfochlorid mit Zinkstaub/Schwefelsäure herzustellen. Die Ausbeute beträgt dabei 72 % der Theorie (Chem. Ber. 86, 179 (1953)). Dieses Verfahren eignet sich wenig für die technische Durchführung, da ein zinkhaltiges Abwasser anfällt. Ferner ist die Reduktion von gegebenenfalls substituierten Benzolsulfochloriden mit Natriumsulfit zu den entsprechenden Natriumbenzolsulfinaten bekannt. In diesen Fällen wird gewöhnlich nach erfolgter Reduktion das Natriumsalz der Sulfinsäure isoliert (siehe FIAT Final Report 949, S. 23 und 24 und DE-A 3 302 647, Beispiel 17). Die Ausbeute an Sulfinat beträgt dabei 80 %. Gemäß dem Verfahren der DE-A 1 816 902 gelingt die Reduktion von aromatischen Sulfochloriden mit Natriumsulfit zu Sulfinsäure sowie die anschließende Reduktion mit SO₂ der angesäuerten Sulfinsäurelösung ohne Zwischenisolierung zu aromatischen Disulfiden mit guten Ausbeuten. Diese Verfahrensweise ist auf die Herstellung von am Ende kristallin abtrennbaren aromatischen Disulfiden ausgerichtet. 4,4'-Difluordiphenyldisulfid ist jedoch ein bei Raumtemperatur flüssiges Produkt und deshalb so nicht herstellbar.

Bei der Herstellung von 4,4'-Difluordiphenyldisulfid ist das Ansäuern der Sulfinatlösung mit einer Dichteerhöhung der Wasserphase verbunden, was dann eine schlecht trennbare Emulsion ergibt. Außerdem wird unnötig viel Salz als Abfall erzeugt.

Bekannt sind auch Hydrierverfahren zur Reduktion von aromatischen Sulfochloriden und aromatischen Disulfiden mit Wasserstoff an Edelmetallkatalysatoren. Nachteilig hierbei sind hohe Wasserstoffdrucke von bis zu 150 bar und Temperaturen bis 150°C (siehe z.B. EP-A 2 755).

Die Reduktion von 4-Chlorbenzolsulfochlorid mit Natriumborhydrid in Tetrahydrofuran führt zu einem Gemisch aus 4,4'-Dichlordiphenyldisulfid und 4-Chlorthiophenol mit einer Ausbeute von ca. 40 %. Pro Mol Sulfochlorid muß ein hoher Überschuß an Natriumborhydrid eingesetzt werden (siehe Chem. Pharm. Bull. 35, 1770 (1987)).

Auch die Reduktion von aromatischen Disulfiden mit Natriumborhydrid ist bekannt. Hierbei ist nach Synth. Commun. 16, 819-825 (1986) ein Lösungsmittelgemisch aus Tetrahydrofuran und Methanol erforderlich, um gute Ausbeuten zu erzielen. Die Verwendung nur eines Lösungsmittels führt zu schlechen Ausbeuten an Thiophenolen. Außerdem ist die bei diesem Verfahren benötigte Natriumborhydrid-Menge mit 2,5 Mol pro Mol Disulfid außerordentlich hoch. Man muß berücksichtigen, daß 1 Mol Natriumborhydrid 8 Reduktionsäquivalente beinhaltet.

Es besteht also nach wie vor das Bedürfnis nach einem technisch einfachen Verfahren zur Herstellung von 4-Fluorthiophenol, bei dem man ausgehend von 4-Fluorbenzolsulfochlorid die Einzelschritte der Synthese mit guten Ausbeuten durchlaufen und die oben geschilderten Nachteile anderer Verfahren weitgehend vermeiden kann.

Es wurde nun gefunden, daß man in vorteilhafter Weise 4-Fluorthiophenol herstellen kann, wenn man 4-Fluorbenzsulfochlorid mit Natriumhydrogensulfitlösung zunächst zu einer 4-Fluorbenzolsulfinsäure-Na-Salzlösung umsetzt, danach diese Lösung mit Schwefeldioxid zu 4,4'-Difluordiphenyldisulfid reduziert und schließlich dieses mit Natriumborhydrid in einem mit Wasser mischbaren inerten organischen Lösungsmittel zu 4-Fluorthiophenol (Na-Salz) umsetzt. Aus dieser Natriumsalz-Lösung läßt sich nach Abdestillieren des Lösungsmittels 4-Fluorthiophenol gewinnen, beispielsweise indem man die Lösung ansäuert, die sich bildende organische Phase abtrennt und gegebenenfalls noch weiter reinigt.

Das erfindungsgemäße Verfahren läßt sich als Eintopf-Synthese durchführen und läßt sich durch folgendes Formelschema illustrieren:

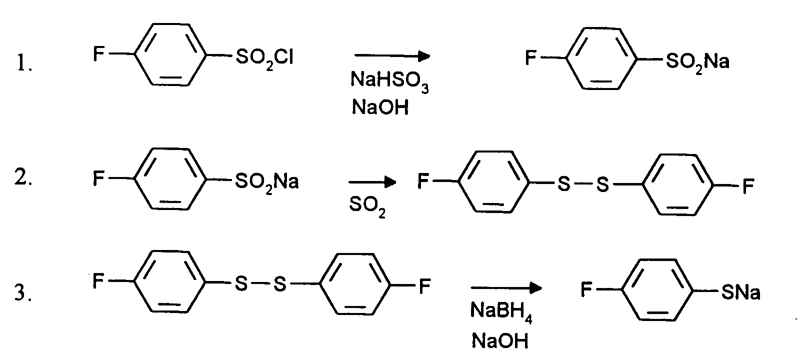

Als Ausgangsmaterial für das Verfahren dient 4-Fluorbenzolsulfochlorid. Das ist z.B. in bekannter Weise herstellbar, etwa durch Sulfochlorierung von Fluorbenzol. Es ist vorteilhaft, in das erfindungsgemäße Verfahren 4-Fluorbenzolsulfochlorid einzusetzen, das von 2-Fluorbenzolsulfochlorid befreit worden ist, z.B. durch fraktionierte Destillation oder fraktionierte Kristallisation.

In der ersten Stufe des erfindungsgemäßen Verfahrens kann man z.B. das 4-Fluorbenzolsulfochlorid in geschmolzener Form (Schmelzpunkt 38°C) in eine vorgelegte wäßrige Lösung von Natriumhydrogensulfit eintragen. Es ist im allgemeinen vorteilhaft, das Natriumhydrogensulfit im Überschuß einzusetzen, z.B. in einem Überschuß von 5 bis 10 Mol-%. Weiterhin ist es vorteilhaft, das Natriumhydrogensulfit in nicht zu konzentrierter Lösung einzusetzen oder Wasser hinzuzufügen. Beispielsweise ist eine 5 bis 20 gew.-%ige Natriumhydrogensulfitlösung gut geeignet. Nach beendeter Zugabe von 4-Fluorbenzolsulfochlorid kann man z.B. noch 1 bis 3 Stunden nachreagieren lassen. Es ist vorteilhaft vor und während der Reaktion den pH-Wert im Bereich 6,3 bis 6,7 zu halten, was beispielsweise durch Zusatz von wäßriger Natronlauge erreicht werden kann. Vorzugsweise wird der pH-Wert im Bereich 6,4 bis 6,6 gehalten. Die Reaktionstemperatur wird vorteilhaft oberhalb des Schmelzpunktes von 4-Fluorbenzolsulfochlorid gewählt, da das Sulfochlorid in kristalliner Form nur sehr langsam reagiert. Beispielsweise kommen Temperaturen im Bereich von 35 bis 80°C, bevorzugt solche im Bereich von 40 bis 80°C, in Frage. Die Reaktion verläuft im allgemeinen mit quantitativem Umsatz und nahezu 100 %iger Selektivität.

Im Hinblick auf die sich anschließende Reaktion der erhaltenen 4-Fluorbenzolsulfinatlösung mit Schwefeldioxid ist ein Ansäuern der Lösung sehr nachteilig. Durch Säurezugabe würde der Druck bei der Reduktion mit Schwefeldioxid unerwünscht erhöht, das spezifische Gewicht der Wasserphase nach beendeter Umsetzung soweit ansteigen, daß eine Phasentrennung von dem flüssig anfallenden 4,4'-Difluordiphenyldisulfid unnötig erschwert wäre und, weil schon bei der Säurezugabe ein zweiphasiges Gemisch entstehen würde, dessen Handhabungsverhalten bei der Folgereaktion ungünstiger ist, als das einer homogenen Lösung.

Für die Umsetzung mit Schwefeldioxid nimmt man also zweckmäßigerweise die 4-Fluorsulfinatlösung wie sie nach der ersten Stufe des erfindungsgemäßen Verfahrens vorliegt. Man kann auch im gleichen Reaktionsgefäß weiterarbeiten.

Es ist im allgemeinen vorteilhaft, Schwefeldioxid in stöchiometrischer Menge oder im Überschuß einzusetzen, z.B. 1,5 bis 1,7 Mol pro Mol 4-Fluorbenzolsulfochlorid. Schwefeldioxid kann in flüssiger Form oder als Gas eingesetzt werden.

Die Reaktionstemperatur während der Reduktion der Sulfinatlösung kann z.B. im Bereich 20 bis 170°C liegen. Vorteilhaft ist es diese Reduktion bei relativ niedriger Temperatur zu beginnen, z.B. bei 20 bis 50°C, und bei relativ höherer Temperatur, z.B. bei 120 bis 160°C, zu Ende zu führen. Es ist häufig vorteilhaft, im Reaktionsgemisch eine relativ höhere Temperatur für 2 bis 10 Stunden beizubehalten. Während dieser Reduktion kann der Druck beispielsweise bis zu 10 bar ansteigen. Vorzugsweise arbeitet man bei Drucken zwischen 1 und 5 bar.

Nach der Reduktion fällt 4,4'-Difluordiphenyldisulfid als gelbes Öl an, z.B. bei ca. 30°C. Die wäßrige (obere) Phase kann leicht von der unteren Disulfid-Phase abgetrennt werden. Es ist vorteilhaft, mit einer nachfolgenden Wäsche mit Wasser das Disulfid von Resten anhaftender Säuren und Salze zu befreien. Die Ausbeute an 4,4'-Difluordiphenyldisulfid beträgt bis zu 98 %, bezogen auf 4-Fluorbenzolsulfochlorid.

Anschließend wird das 4,4'-Difluordiphenyldisulfid in einem mit Wasser mischbaren inerten organischen Lösungsmittel zu 4-Fluorthiophenol (Na-Salz) umgesetzt. Dazu kann man z.B. das im Reaktionsbehälter befindliche Disulfid in einem mit Wasser mischbaren, gegen Natriumborhydrid inerten organischen Lösungsmittel lösen und eine wäßrige, Natronlauge enthaltende Lösung von Natriumborhydrid in dem Maße zugeben, daß die gewünschte Reaktionstemperatur eingehalten wird. Die Reaktionstemperatur kann z.B. zwischen der Raumtemperatur und dem Siedepunkt des jeweiligen Lösungsmittels bzw. dem Siedepunkt des Azeotrops aus Wasser und dem jeweiligen Lösungsmittel liegen. Bevorzugt läßt man die Reaktion unter Rückflußsieden ablaufen, um die Reaktionswärme auf diese Weise abführen zu können. Temperaturen im Bereich 60 bis 100°C sind bevorzugt. Man kann pro Mol 4,4'-Difluordiphenyldisulfid beispielsweise 1,8 bis 2,2 Mole NaOH einsetzen.

Nach beendeter Zugabe des Natriumborhydrids ist es vorteilhaft noch 1 bis 5 Stunden nachzurühren. Dann liegt üblicherweise eine klare und praktisch farblose Lösung vor, die 4-Fluorthiophenol als Na-Salz enthält. Diese Lösung kann man z.B. aufarbeiten, indem man mit Mineralsäuren, bevorzugt mit Salzsäure ansäuert, die sich bildende untere 4-Fluorthiophenol-Phase abtrennt und nach dem Entfernen von verunreinigenden Salzen durch Waschen mit Wasser und durch Andestillieren oder vollständige Destillation weiter reinigt. Man kann so 4-Fluorthiophenol z.B. in Reinheiten von über 99 % und in Ausbeuten bis zu 88 % (bezogen auf 4,4'-Difluordiphenyldisulfid) erhalten.

Natriumborhydrid kann man z.B. in Mengen von 0,25 bis 1,0 Mol pro Mol des Disulfids einsetzen. Vorzugsweise verwendet man einen Überschuß an Natriumborhydrid, beispielsweise 0,3 bis 0,6 Mol pro Mol des Disulfids. Größere Überschüsse sind aus technischer Sicht nicht vorteilhaft und aus ökonomischen Erwägungen nicht sinnvoll.

Als inerte, mit Wasser mischbare organische Lösungsmittel kommen beispielsweise niedere aliphatische Alkohole wie Methanol, Ethanol und Isopropanol sowie Tetrahydrofuran, Diglyme und Dimethylformamid in Frage. Bevorzugt sind Lösungsmittel, die sich leicht von der wäßrigen Lösung abtrennen lassen, z.B. durch Destillation. Besonders bevorzugt setzt man Methanol oder Isopropanol ein.

Die Lösungsmittelmenge wird vorteilhafterweise so bemessen, daß während der Natriumborhydrid-Zugabe und während des gesamten Reduktionsvorgangs das Reaktionsgemisch eine homogene Phase bildet.

Das bei der Reduktion mit Natriumborhydrid verwendete Lösungsmittel kann nach dem Abdestillieren (rein oder als Azeotrop mit Wasser) wiederverwendet werden.

Durch Ansäuern der wäßrigen Natrium-4-fluorthiophenolat-Lösung, z.B. mit konzentrierter Salzsäure bis zu einem pH-Wert von 0,5 bis 2,5, kann man 4-Fluorthiophenol als klare untere Phase erhalten und abtrennen. Durch Andestillieren kann man sehr reines 4-Fluorthiophenol als klare, farblose Flüssigkeit erhalten.

Der Gehalt des isolierten 4-Fluorthiophenols liegt häufig über 99 %. Eine Verunreinigung durch 2-Fluorthiophenol läßt sich dadurch vermeiden, daß man in die Reduktion von 4-Fluorbenzolsulfochlorid ein Material einsetzt, aus dem das 2-Isomere abgetrennt worden ist.

4-Fluorthiophenol ist ein wichtiges Zwischenprodukt, z.B. zur Herstellung pharmazeutischer Wirksubstanzen (siehe EP-A 100 172).

Die folgenden Beispiele erläutern die Erfindung.

### Beispiele

### Beispiel 1

345 ml wäßrige Natriumhydrogensulfitlösung (40 %ig) und 1 050 ml Wasser wurden in einem Druckbehälter unter Stickstoff vorgelegt und mit 56 ml Natronlauge (44,8 %ig) auf einen pH-Wert von 6,5 eingestellt. Währenddessen wurde das Gemisch auf 40°C erwärmt. Danach ließ man 315,8 g 4-Fluorbenzolsulfochlorid bei 40 bis 45°C zutropfen und hielt durch gleichzeitiges Zutropfen von 225 ml Natronlauge (44,8 %ig) den pH-Wert bei 6,5. Nach beendeter Zugabe des 4-Fluorbenzolsulfochlorids wurde das Gemisch noch 2 Stunden nachgerührt.

Nach zweimaligem Spülen mit Stickstoff wurden bei Raumtemperatur 118 ml Schwefeldioxid in flüssiger Form in den Druckbehälter gedrückt. Das Gemisch wurde unter Rühren innerhalb von 3 Stunden auf 135°C erhitzt und anschließend 5 Stunden bei dieser Temperatur nachgerührt. Der Druck betrug während der Reaktion ca. 5 bar.

Nach beendeter Reaktion wurde das Gemisch auf 30°C abgekühlt, der Druckbehälter entspannt, die wäßrige Phase abgetrennt und die verbleibende Disulfid-Phase mit Wasser gewaschen. Die Ausbeute an 4,4'-Difluordiphenyldisulfid betrug 98 %, bezogen auf eingesetztes 4-Fluorbenzolsulfochlorid.

### Beispiel 2

187,4 g 4,4'-Difluordiphenyldisulfid wurden in 650 ml Isopropanol und 90 ml Wasser gelöst. Unter Stickstoff wurde die Lösung auf Rückflußtemperatur erhitzt (80 bis 82°C) und bei dieser Temperatur eine Lösung von 15,7 g NaBH₄ (98 %ig) in wäßriger Natronlauge im Verlauf einer Stunde dem Gemisch zugetropft. Die Natronlauge war erhalten worden aus 92 ml 45 %iger Natronlauge und 400 ml Wasser. Das Reaktionsgemisch wurde 2 Stunden nachgerührt. Danach war die anfangs gelbe Färbung völlig verschwunden. Nun wurde das Isopropanol vollständig abdestilliert, gleichzeitig wurden 700 ml Wasser hinzugefügt. Die Lösung wurde auf 60°C abgekühlt und durch Zugabe von konzentrierter Salzsäure (ca. 160 ml) auf einen pH-Wert von 1 eingestellt, wobei sich eine untere Phase abschied, die im wesentlichen 4-Fluorthiophenol enthielt. Durch Andestillieren der abgetrennten unteren Phase wurde ein wasserfreies, klares 4-Fluorthiophenol (Reinheit: 98,7 %) in einer Ausbeute von 74 % der Theorie erhalten.

### Beispiel 3

186,4 g 4,4'-Difluordiphenyldisulfid wurden in 325 ml Isopropanol und 45 ml Wasser gelöst und anschließend wie in Beispiel 2 beschrieben mit einer NaBH₄-Lösung reduziert und das Reaktionsgemisch wie dort beschrieben aufgearbeitet. Es wurde 99,2 % reines 4-Fluorthiophenol in einer Ausbeute von 94,3 % der Theorie erhalten. Durch Extraktion der Wasserphase ließen sich weitere 4,2 % 4-Fluorthiophenol gewinnen, was dann eine Gesamtausbeute von 98,5 % ergab.

### Beispiel 4

195 g 4,4'-Difluordiphenyldisulfid wurden in 650 ml Methanol und 90 ml Wasser gelöst und durch Zutropfen einer Lösung von 16,3 g NaBH₄ in 370 ml Wasser und 140 g einer 43,8 %igen wäßrigen Natronlauge bei 70 bis 78°C in analoger Weise wie in Beispiel 2 beschrieben reduziert. Die Aufarbeitung und Isolierung erfolgte ebenfalls analog zu Beispiel 2. 4-Fluorthiophenol wurde in einer Reinheit von 99,5 % und in einer Ausbeute von 95 % der Theorie erhalten. Durch Extraktion der Wasserphase konnten noch weitere 3,2 % 4-Fluorthiophenol gewonnen werden, so daß die Gesamtausbeute 98,2 % betrug.

### Beispiel 5

Die Reduktion von 190,8 g 4,4'-Difluordiphenyldisulfid wurde entsprechend Beispiel 4 durchgeführt, jedoch anstelle von Methanol 650 ml Tetrahydrofuran eingesetzt. 4-Fluorthiophenol wurde in einer Ausbeute von 95,1 % der Theorie erhalten, durch Extraktion der Wasserphase weitere 3,1 %, so daß die Gesamtausbeute 98,5 % betrug. Die Reinheit des Produkts war 99,6 %.

### Beispiel 6

194 g 4,4'-Difluordiphenyldisulfid wurden in 650 ml Isopropanol und 90 ml Wasser vorgelegt und durch Zutropfen einer Lösung von 16,2 g NaBH₄ in 370 ml Wasser und 139,3 g 43,8 %iger wäßriger Natronlauge (94,8 ml) bei 50°C innerhalb von 2 Stunden und Nachrühren des sich bildenden Gemisches während weiterer 10 Stunden und Stehenlassen der Lösung über Nacht reduziert. Die Aufarbeitung und Isolierung des 4-Fluorthiophenols erfolgte wie in Beispiel 2 beschrieben. 4-Fluorthiophenol wurde in einer Ausbeute von 93,2 % der Theorie erhalten, durch Extraktion weitere 6,5 g, so daß die Gesamtausbeute 96,5 % betrug. Die Reinheit des Produkts betrug 99,4 %

## Patentansprüche

1. Verfahren zur Herstellung von 4-Fluorthiophenol, dadurch gekennzeichnet, daß man 4-Fluorbenzolsulfochlorid mit Natriumhydrogensulfitlösung zunächst zu einer 4-Fluorbenzolsulfinsäure-Na-Salzlösung umsetzt, danach diese mit Schwefeldioxid zu 4,4'-Difluordiphenyldisulfid reduziert und schließlich dieses mit Natriumborhydrid in einem mit Wasser mischbaren inerten organischen Lösungsmittel zu 4-Fluorthiophenol (Na-Salz) umsetzt.

2. Verfahren nach Anspruch 1, dadurch gekennzeichnet, daß man 4-Fluorbenzolsulfochlorid einsetzt, das von 2-Fluorbenzolsulfochlorid befreit worden ist.

3. Verfahren nach Ansprüchen 1 und 2, dadurch gekennzeichnet, daß man 4-Fluorbenzolsulfochlorid in geschmolzener Form bei 35 bis 80°C in eine vorgelegte wäßrige Natriumhydrogensulfit-Lösung einträgt, den pH-Wert im Bereich 6,3 bis 6,7 hält und nach beendeter Zugabe des 4-Fluorbenzolssulfochlorids noch 1 bis 3 Stunden nachreagieren läßt.

4. Verfahren nach Ansprüchen 1 bis 3, dadurch gekennzeichnet, daß man das Natriumhydrogensulfit in einem Überschuß von 5 bis 10 Mol-% einsetzt.

5. Verfahren nach Ansprüchen 1 bis 4, dadurch gekennzeichnet, daß man die in der ersten Stufe erhaltene Lösung von 4-Fluorbenzolsulfinat im gleichen Reaktionsgefäß mit 1,5 bis 1,7 Mol Schwefeldioxid pro Mol 4-Fluorbenzolsulfochlorid bei 20 bis 170°C bei einem Druck bis zu 10 bar innerhalb von 2 bis 10 Stunden umsetzt.

6. Verfahren nach Ansprüchen 1 bis 5, dadurch gekennzeichnet, daß man das nach der Reduktion mit Schwefeldioxid erhaltene 4,4'-Difluordiphenyldisulfid mit Wasser wäscht und dann in einem mit Wasser mischbaren, gegen Natriumborhydrid inerten organischen Lösungsmittel durch Zugabe einer wäßrigen, Natronlauge enthaltenden Lösung von Natriumborhydrid zu 4-Fluorthiophenol (Na-Salz) umsetzt.

7. Verfahren nach Anspruch 6, dadurch gekennzeichnet, daß man pro Mol 4,4'-Difluordiphenyldisulfid 1,8 bis 2,2 Mole NaOH und 0,25 bis 1,0 Mol Natriumborhydrid einsetzt und die Reaktion bei einer Temperatur im Bereich 60 bis 100°C durchführt.

8. Verfahren nach Ansprüchen 1 bis 7, dadurch gekennzeichnet, daß man als mit Wasser mischbare inerte organische Lösungsmittel niedrige aliphatische Alkohole, Tetrahydrofuran, Diglyme oder Dimethylformamid einsetzt.

9. Verfahren nach Ansprüchen 1 bis 8, dadurch gekennzeichnet, daß man nach der Reduktion mit Natriumborhydrid das Lösungsmittel abdestilliert und durch Ansäuern einer wäßrigen Natrium-4-fluorthiophenolat-Lösung 4-Fluorthiophenol erhält.

10. Verfahren nach Anspruch 9, dadurch gekennzeichnet, daß man das erhaltene 4-Fluorthiophenol durch Andestillieren weiter reinigt.

## Claims

1. Process for the preparation of 4-fluorothiophenol, characterized in that 4-fluorobenzenesulphonyl chloride is first reacted with sodium hydrogen sulphite solution to give a solution of sodium 4-fluorobenzenesulphinate, this is then reduced with sulphur dioxide to give 4,4'-difluorodiphenyl disulphide and finally this is reacted with sodium borohydride in a water-miscible inert organic solvent to give 4-fluorothiophenol (sodium salt).

2. Process according to Claim 1, characterized in that 4-fluorobenzenesulphonyl chloride is used which has been freed from 2-fluorobenzenesulphonyl chloride.

3. Process according to Claims 1 and 2, characterized in that 4-fluorobenzenesulphonyl chloride is introduced in molten form at 35 to 80°C into a given aqueous sodium hydrogen sulphite solution, the pH is kept in the range from 6.3 to 6.7 and when addition of the 4-fluorobenzenesulphonyl chloride is complete, the mixture is allowed to continue reacting for a further 1 to 3 hours.

4. Process according to Claims 1 to 3, characterized in that the sodium hydrogen sulphite is used in an excess of 5 to 10 mol%.

5. Process according to Claims 1 to 4, characterized in that the solution of 4-fluorobenzenesulphinate obtained in the first stage is reacted in the same reaction vessel with 1.5 to 1.7 mol of sulphur dioxide per mol of 4-fluorobenzenesulphonyl chloride at 20 to 170°C at a pressure up to 10 bar in the course of 2 to 10 hours.

6. Process according to Claims 1 to 5, characterized in that the 4,4'-difluorodiphenyl disulphide obtained after the reduction with sulphur dioxide is washed with water and then, in a water-miscible organic solvent inert towards sodium borohydride, is converted to 4-fluorothiophenol (sodium salt) by addition of an aqueous sodium hydroxide-containing solution of sodium borohydride.

7. Process according to Claim 6, characterized in that 1.8 to 2.2 mol of NaOH and 0.25 to 1.0 mol of sodium borohydride are used per mol of 4,4'-difluorodiphenyl disulphide and the reaction is carried out at a temperature in the range from 60 to 100°C.

8. Process according to Claims 1 to 7, characterized in that the water-miscible inert organic solvent used is low aliphatic alcohols, tetrahydrofuran, diglyme or dimethylformamide.

9. Process according to Claims 1 to 8, characterized in that after the reduction with sodium borohydride the solvent is distilled off and by acidification of an aqueous sodium 4-fluorothiophenolate solution 4-fluorothiophenol is obtained.

10. Process according to Claim 9, characterized in that the 4-fluorothiophenol obtained is further purified by incipient distillation.

## Revendications

1. Procédé pour la préparation du 4-fluorothiophénol, caractérisé en ce qu'on fait réagir, dans un premier temps, du sulfochlorure de 4-fluorobenzène avec une solution d'hydrogénosulfite de sodium pour obtenir une solution de sel de sodium de l'acide 4-fluorobenzènesulfinique, ensuite on réduit cette dernière avec du dioxyde de soufre pour obtenir du disulfure de 4,4'-difluorodiphényle et enfin, on fait réagir ce dernier avec du borohydrure de sodium dans un solvant organique inerte miscible à l'eau pour obtenir le 4-fluorothiophénol (sel de sodium).

2. Procédé selon la revendication 1, caractérisé en ce qu'on met en oeuvre du sulfochlorure de 4-fluorobenzène qui a été libéré du sulfochlorure de 2-fluorobenzène.

3. Procédé selon les revendications 1 et 2, caractérisé en ce qu'on introduit le sulfochlorure de 4-fluorobenzène à l'état fondu à une température de 35 à 80°C dans une solution aqueuse d'hydrogénosulfite de sodium déposée au préalable, on maintient la valeur de pH dans le domaine de 6,3 à 6,7 et, au terme de l'addition du sulfochlorure de 4-fluorobenzène, on laisse encore réagir ultérieurement pendant un laps de temps de 1 à 3 heures.

4. Procédé selon les revendications 1 à 3, caractérisé en ce qu'on met en oeuvre l'hydrogénosulfite de sodium en un excès de 5 à 10 moles %.

5. Procédé selon les revendications 1 à 4, caractérisé en ce qu'on fait réagir la solution de sulfinate de 4-fluorobenzène obtenue à la première étape dans le même récipient réactionnel avec de 1,5 à 1,7 mole de dioxyde de soufre par mole de sulfochlorure de 4-fluorobenzène, à une température de 20 à 170°C, sous une pression allant jusqu'à 10 bar, pendant un laps de temps de 2 à 10 heures.

6. Procédé selon les revendications 1 à 5, caractérisé en ce qu'on lave avec de l'eau le disulfure de 4,4'-difluorodiphényle obtenu après la réduction avec le dioxyde de soufre et on le fait ensuite réagir dans un solvant organique inerte vis-à-vis du borohydrure de sodium et miscible à l'eau par addition d'une solution aqueuse de borohydrure de sodium contenant de la lessive de soude pour obtenir le 4-fluorothiophénol (sel de sodium).

7. Procédé selon la revendication 6, caractérisé en ce qu'on met en oeuvre, par mole du disulfure de 4,4'-difluorodiphényle, de 1,8 à 2,2 moles de NaOH et de 0,25 à 1,0 mole de borohydrure de sodium, et on effectue la réaction à une température dans le domaine de 60 à 100°C.

8. Procédé selon les revendications 1 à 7, caractérisé en ce qu'on met en oeuvre, comme solvant inorganique inerte miscible à l'eau, des alcools aliphatiques inférieurs, le tétrahydrofuranne, le diglyme ou le diméthylformamide.

9. Procédé selon les revendications 1 à 8, caractérisé en ce qu'on sépare le solvant par distillation après la réduction avec le borohydrure de sodium et on obtient, par acidification d'une solution aqueuse de 4-fluorothiophénolate de sodium, le 4-fluorothiophénol.

10. Procédé selon la revendication 9, caractérisé en ce qu'on soumet le 4-fluorothiophénol obtenu à une purification ultérieure par distillation.
